# EUROPEAN PATENT APPLICATION

(11) **EP 3 838 909 A1**
(43) Date of publication of application: **23.06.2021**
(21) Application number: 19216405.1
(22) Date of filing: 16.12.2019
(51) Int. Cl.: C07H 1/00, C07H 19/056, C07H 17/02

(54) **LARGE SCALE PROCESS FOR THE PREPARATION OF 5-BROMOPYRIDIN-3-YL-3-DEOXY-3-[4-(3,4,5-TRIFLUOROPHENYL)-1H-1,2,3-TRIAZOL-1-YL]- 1-THIO-ALPHA-D-GALACTOPYRANOSIDE**

(71) Applicant: Galecto Biotech AB, 2200 Copenhagen (DK)
(72) Inventor: LISE, Gravelle, 2200 COPENHAGEN (DK); TYRELL, Andrew, Bristol, BS2 0ZX (GB); KINNAERT, Christine, Bristol, BS2 0ZX (GB); ZETTERBERG, Frederik, 2200 COPENHAGEN (DK); WEYMOUTH-WILSON, Alexander, Reading RG2 9LH (GB); CLARKSON, Robert, Reading RG2 9LH (GB)
(74) Representative: Kjerrumgaard, Lars Bo

(57) **Abstract**

The present invention relates to a process for preparing a compound of formula (I) 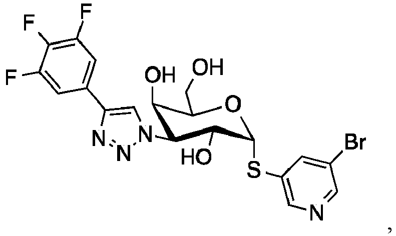 wherein said process is suitable for large scale synthesis.

## Description

### Technical field

The present invention relates to a process of preparing 5-Bromopyridin-3-yl 3-de-oxy-3-[4-(3,4,5-trifluorophenyl)-1*H*-1,2,3-triazol-1-yl]-1-thio-α-D-galactopyranoside which process can be scaled up. The process parameters are stable, and the process is suitable for GMP.

### Background Art

The compound of formula I has been described in international patent application publication number WO2016120403 as a galectin 3 inhibitor useful for treating various disorders or diseases, as described therein. The compound of formula I was made in a 60% yield in a small scale lab process, but no parameters for scaling up has been disclosed.

### Summary of the invention

The present invention relates to a new process for preparing 5-Bromopyridin-3-yl 3-deoxy-3-[4-(3,4,5-trifluorophenyl)-1*H*-1,2,3-triazol-1-yl]-1-thio-α-D-galactopyranoside which process can be scaled up to large scale and/or industrial scale such as 30 kg or higher. The process can also be used for smaller scale such as from 200 g to 3 kg.

In a first aspect the present invention relates to a process, such as suitable for large scale synthesis, for preparing 5-Bromopyridin-3-yl 3-deoxy-3-[4-(3,4,5-trifluorophenyl)-1H-1,2,3-triazol-1-yl]-1-thio-α-D-galactopyranoside having formula (I) 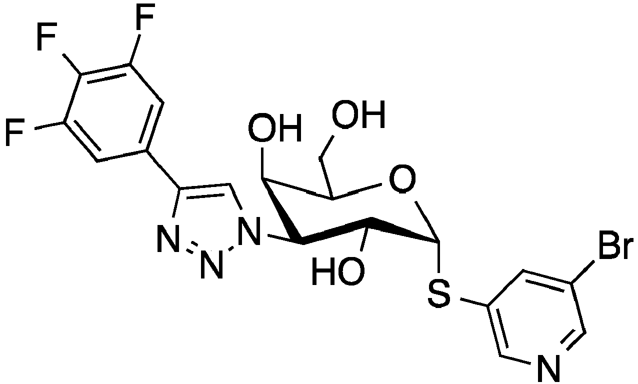 wherein the process comprises the consecutive steps of
a) reacting a compound of formula IX 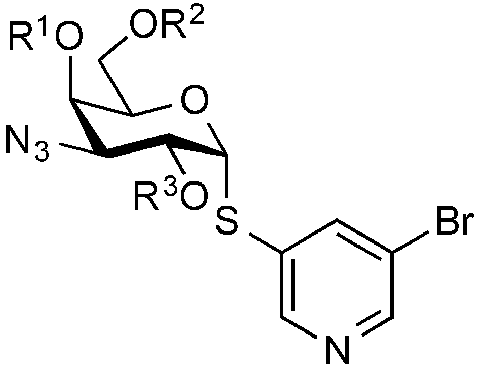 wherein R1, R2, and R3 are independently selected from protecting groups or hydrogen, provided that at least one of R1, R2, and R3 is a protecting group, with 5-ethynyl-1,2,3-trifluorobenzene) or a silane protected 5-ethynyl-1,2,3-trifluorobenzene), and a catalyst and optionally adding a base in an organic solvent, and optionally adding a basic fluoride source agent, such as TBAF, under suitable conditions to obtain a compound of formula X 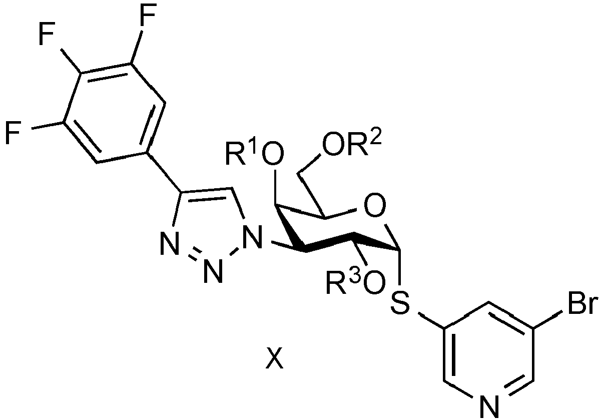 wherein R1, R2, R3 are as defined above, and
b) removing the protecting groups of the compound of formula X to obtain the compound of formula I. In an embodiment the compound of formula I is obtained as a solid product, such as a crystalline or amorphous product.

In one embodiment the basic fluoride source agent is added. In another embodiment no basic fluoride source agent is added.

In an embodiment the compound of formula I is isolated as a crystalline form, such as a polymorph, e.g. polymorphic form 1.

In another embodiment the compound of formula I is isolated as a salt, such as a sulfate, bromide or phosphate salt, preferably a HCl salt. Typically, a crystalline HCl salt.

In a further embodiment the suitable conditions in step a) are reacting a compound of formula IX wherein R1, R2, R3 are independently selected from protecting groups or hydrogen, provided that at least one of R1, R2, R3, is a protecting group, with trimethyl((3,4,5-trifluorophenyl)ethynyl)silane in the organic solvent at a suitable temperature, optionally under inert atmosphere, and adding a catalyst and optionally adding a base in the organic solvent to create a reaction mixture and optionally heating the reaction mixture to raise the temperature at least 15 °C above the suitable temperature, and adding the basic fluoride source agent and continue the reaction for at least 1 hour to obtain the compound of formula X wherein R1, R2, R3 are as defined above.

In a still further embodiment R1, R2, R3 are independently selected from ester protecting groups, such as acetyl, benzoyl and pivaloyl, typically all R1, R2, R3, are acetyl.

The compound X may be further purified and isolated as a solid. Typically, compound X is isolated whereas purification takes place later in the process as majority of impurities are intermediates in the deacetylation to compound of formula I.

In a further embodiment the reaction takes place under inert atmosphere, such as argon or nitrogen atmosphere.

In a still further embodiment the organic solvent is selected from toluene or a polar aprotic solvent, such as acetonitrile or DMF, and mixtures thereof.

In a further embodiment the suitable temperature is between 15 and 25 °C, such as about room temperature.

In a still further embodiment the temperature is raised in the reaction mixture heating the mixture to 45 °C to 70 °C, such as about 60 °C.

In a further embodiment the reaction is continued for at least 2 hours, such as 3 hours, e.g. from 2.5 to 4 hours.

In a still further embodiment the catalyst is a metal catalyst, such as a metal halide, e.g. Cu(I) or Cu(II), in particular Cu halide, such as Cu iodide.

In a further embodiment the base is an organic base, such as triethylamine or DIPEA.

In a still further embodiment the basic fluoride source agent is TBAF.

In a still further embodiment the molar ratio between the compound of formula IX and trimethyl((3,4,5-trifluorophenyl)ethynyl)silane is 5:4 to 1:3, such as 1:1 to 5:7, typically 5:6, and the organic solvent is in surplus.

In a further embodiment the molar ratio between the compound of formula IX and the catalyst is 20:1 to 2:1, such as 20:1 to 5:1, typically 10:1 and the organic solvent is in surplus.

In a still further embodiment the molar ratio between the compound of formula IX and the base is 1:1 to 1:10, such as 2:3 to 1:3, typically 1:2 and the organic solvent is in surplus.

In a further embodiment the removing of protecting groups in step b) is done by mixing the compound of formula X in an organic solvent and with a base optionally under inert atmosphere and reacting for at least 15 minutes at a suitable temperature, followed by washing with an ether to obtain the compound of formula I. Typically, the ether is *tert-*Butylmethyl ether (TBME). Preferably, the suitable temperature is 15-25 °C, such as about room temperature. Typically, the organic solvent is selected from an alcohol, such as C₁₋₆ alkohol, e.g. methanol. Furthermore, the base is preferably selected from a base, such as an organic base, in a concentration sufficient to provide a pH of 12 or higher. Typically, the base is sodium methoxide in methanol, such as 25 wt% sodium methoxide solution in methanol.

In an embodiment the reaction with a base is for at least 1 hour, such as 2-24 hours.

In a further embodiment the present process of the invention comprises a step directly preceding step a)
(ia) reacting a compound of formula VIII 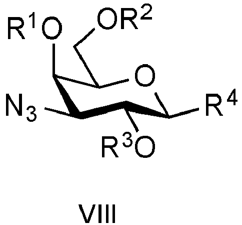 wherein R1, R2, and R3 are independently selected from protecting groups or hydrogen, provided that at least one of R1, R2 and R3 is a protecting group, and R4 is a halogen, with 5-bromopyridine-3-thiol and a base in a suitable organic solvent under suitable conditions, optionally under inert atmosphere, to obtain the compound of formula IX wherein R1, R2, and R3 are independently selected from protecting groups or hydrogen, provided that at least one of R1, R2, and R3 is a protecting group. The base may be selected from NaH, KOtBu, KOH or sodium bis(trimethylsilyl)amide or carbonate bases, e.g. K₂CO₃ and/or Cs₂CO₃. In an embodiment the base is selected from NaH, KOtBu, KOH, sodium bis(trimethylsilyl)amide, K₂CO₃ and/or Cs₂CO₃.

In an embodiment the compound of formula IX is obtained as a solid.

In a further embodiment the deprotonating agent is sodium bis(trimethylsilyl)amide.

In a still further embodiment R1, R2, R3 are all acetyl groups and R4 is as defined above. Preferably, R4 is chlorine.

In a further embodiment the reaction takes place under inert atmosphere. Typically, under an argon or nitrogen atmosphere.

In a still further embodiment the organic solvent is selected from the group consisting of ethyl acetate, THF, toluene, DMF and acetonitrile, and mixtures thereof.

In a further embodiment the suitable conditions in step (ia) are reacting a compound of formula VIII wherein R1, R2, R3 and R4, are as defined above, optionally under inert atmosphere and at a suitable temperature with 5-bromopyridine-3-thiol and the base in an organic solvent, and maintaining the reaction mixture at the suitable temperature, then continue the reaction for at least 15 minutes, and optionally isolating and purifying to obtain the compound of formula IX as a solid. Preferably, the base is cooled to below room temperature before adding 5-bromopyridine-3-thiol over a suitable time and at a suitable temperature and followed by addition of the compound of formula VIII.

In a still further embodiment the suitable temperature is below 25 °C.

In a further embodiment the reaction is continued for at least 2 hours, such as 16-72 hours, at the suitable temperature.

In a still further embodiment the molar ratio between the compound of formula VIII and the base is 1:1 to 1:3, such as 5:7.

In a still further embodiment the process of the present invention comprises a step directly preceding step ia)
(ib) reacting a compound of formula VII 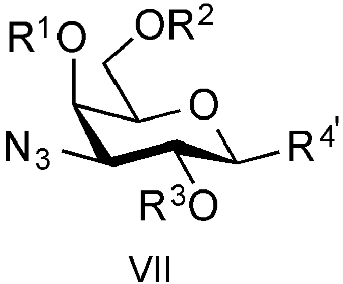 wherein R1, R2, and R3 are independently selected from protecting groups or hydrogen, provided that at least one of R1, R2 and R3 is a protecting group, and R4' is a SR5 or OR5 wherein R5 is selected from H, Z"-C₁₋₆ alkyl, Z"-C₁₋₆ alkenyl, Z"-C₃₋₆ branched alkyl, Z"-C₃₋₆ cyclo alkyl Z"-heteroaryl and Z"-aryl wherein Z" is SO, SO₂, C=O or C=S, with a reagent for activating the anomeric position for nucleophilic substitution, such as a halogenating agent, in a suitable organic solvent, with a suitable catalyst, optionally under inert atmosphere, under suitable conditions to obtain the compound of formula VIII wherein R1, R2, and R3 are independently selected from protecting groups or hydrogen, provided that at least one of R1, R2, and R3 is a protecting group and R4 is a halogen.

In an embodiment the reaction takes place under an inert atmosphere, such as an argon or nitrogen atmosphere.

In a further embodiment the organic solvent is an aprotic solvent, preferably dichloromethane, toluene or α,α,α-trifluorotoluene, and mixtures thereof.

In a further embodiment the reagent for activating the anomeric position for nucleophilic substitution is a halogenating agent. Typically, the halogenating agent is a metal halide, for example, AlCl₃, or SOCl₂, dichloromethyl methyl ether (DCMME) or a halide of phosphorus. Preferably the halogenating agent is PCl₅.

In a further embodiment the catalyst is an acid, such as a lewis acid, preferably BF₃·OEt₂.

In a still further embodiment the suitable conditions involve a suitable temperature of between 15 and 45 °C. In a further embodiment the reaction is continued for at least 15 minutes, at least ½ hour, such as 12-96 hours, at the suitable temperature.

In a still further embodiment the molar ratio between the compound of formula VII and the reagent for activating the anomeric position for nucleophilic substitution such as the halogenating agent, is 5:1 to 1:5, typically 5:6.

In a further embodiment the molar ratio between the compound of formula VII and the catalyst is 10:1 to 200:1, typically 100:1.

In a still further aspect the present invention relates to a process of preparing a compound of formula III as well as formula IV starting from compound of formula II.

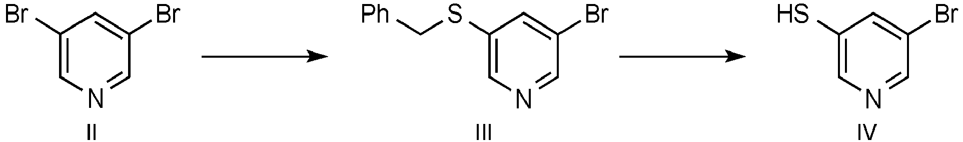

In a further aspect the present invention relates to preparing a compound of formula VI starting from compound of formula V.

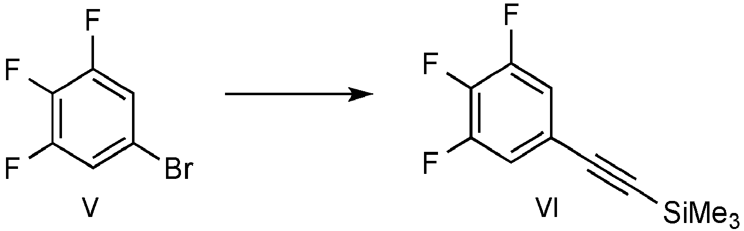

### Detailed Description

The compound of formula (I) has the chemical name (IUPAC) 5-Bromopyridin-3-yl 3-deoxy-3-[4-(3,4,5-trifluorophenyl)-1*H*-1,2,3-triazol-1-yl]-1-thio-α-D-galactopyranoside and may be prepared as described in WO2016120403. The yield is relatively low and the process in not directly possible to scale up.

Moreover, throughout the application the terms "the compound of formula I" or "the compound having formula I" or "5-Bromopyridin-3-yl 3-deoxy-3-[4-(3,4,5-trifluorophenyl)-1*H*-1,2,3-triazol-1-yl]-1-thio-α-D-galactopyranoside having formula I" are used interchangeable and means the compound of formula I in any solid form or liquid form, such as a crystalline form, in particular a polymorphic form, or amorphous form, and furthermore is intended to comprise the free form, any solvate or any salt thereof.

Consequently, a new process has been developed concerning a process, such as suitable for large scale synthesis, for preparing 5-Bromopyridin-3-yl 3-deoxy-3-[4-(3,4,5-trifluorophenyl)-1H-1,2,3-triazol-1-yl]-1-thio-α-D-galactopyranoside having formula (I) 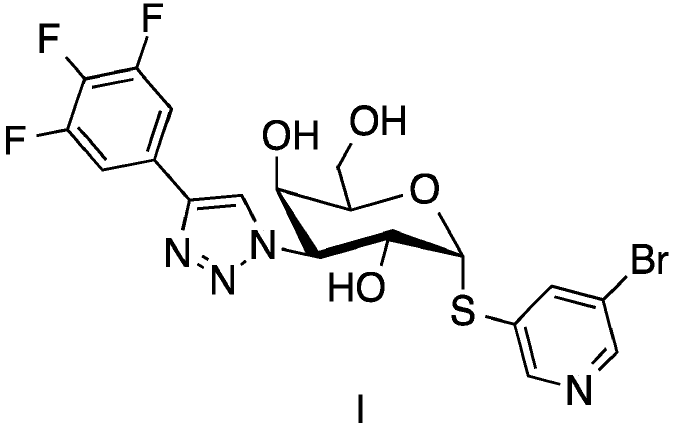 wherein the process comprises the consecutive steps of
a) reacting a compound of formula IX 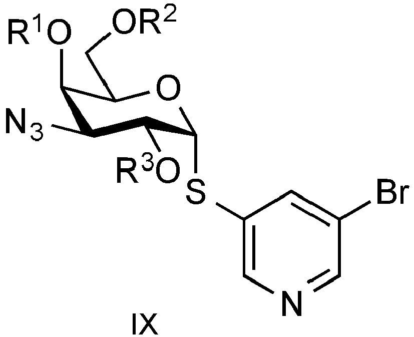 wherein R1, R2, and R3 are independently selected from protecting groups or hydrogen, provided that at least one of R1, R2, and R3 is a protecting group, with 5-ethynyl-1,2,3-trifluorobenzene) or a silane protected 5-ethynyl-1,2,3-trifluorobenzene), such as trimethyl((3,4,5-trifluorophenyl)ethynyl)silane, and a catalyst and optionally adding a base in an organic solvent, and optionally adding a basic fluoride source agent, such as TBAF, under suitable conditions to obtain a compound of formula X 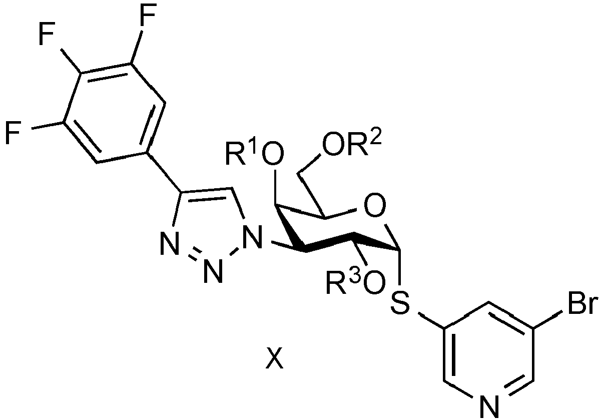 wherein R1, R2, R3 are as defined above, and
b) removing the protecting groups of the compound of formula X to obtain the compound of formula I.

In an embodiment the compound of formula I is obtained as a solid product, such as a crystalline or amorphous product. In an embodiment the compound of formula I is isolated as a crystal, such as a polymorph. Preferably, the compound of formula I is isolated as the polymorphic form 1. In another embodiment the compound of formula I is isolated as a salt, such as a HCl salt. Typically, a crystalline HCl salt.

In a further embodiment the suitable conditions in step a) are reacting a compound of formula IX wherein R1, R2, R3 are acetyl groups or hydrogen, provided that at least one of R1, R2, R3, is an acetyl group, with a silane protected 5-ethynyl-1,2,3-trifluorobenzene in toluene or a polar aprotic solvent, and mixtures thereof, at a suitable temperature between 15 and 25 °C, optionally under inert atmosphere, and adding a catalyst and optionally a base in the organic solvent to create a reaction mixture and optionally heating the reaction mixture to raise the temperature at least 15 °C above the suitable temperature, and adding the basic fluoride source agent and continue the reaction for at least 1 hour to obtain the compound of formula X wherein R1, R2, R3 are as defined above.

In a still further embodiment the suitable conditions in step a) are reacting a compound of formula IX wherein R1, R2, R3 are acetyl groups or hydrogen, provided that at least one of R1, R2, R3, is an acetyl group, with 5-ethynyl-1,2,3-trifluorobenzene in toluene or a polar aprotic solvent, and mixtures thereof, at a suitable temperature between 15 and 25 °C, optionally under inert atmosphere, and adding a catalyst and optionally a base in the organic solvent to create a reaction mixture and optionally heating the reaction mixture to raise the temperature at least 15 °C above the suitable temperature, and continue the reaction for at least 1 hour to obtain the compound of formula X wherein R1, R2, R3 are as defined above.

In a further embodiment the suitable conditions in step a) are reacting a compound of formula IX wherein R1, R2, R3 are acetyl groups or hydrogen, provided that at least one of R1, R2, R3, is an acetyl group, with trimethyl((3,4,5-trifluorophenyl)ethynyl)silane in toluene or a polar aprotic solvent, and mixtures thereof, at a suitable temperature between 15 and 25 °C, optionally under inert atmosphere, and adding a catalyst and optionally a base in the organic solvent to create a reaction mixture and optionally heating the reaction mixture to raise the temperature at least 15 °C above the suitable temperature, and adding the basic fluoride source agent and continue the reaction for at least 1 hour to obtain the compound of formula X wherein R1, R2, R3 are as defined above.

In a still further embodiment R1, R2, R3 are independently selected form ester protecting groups, such as acetyl, benzoyl and pivaloyl, typically all R1, R2, R3, are acetyl.

The compound X may be further purified and isolated as a solid. Typically, compound X is isolated whereas purification takes place later in the process as majority of impurities are intermediates in the deacetylation to compound of formula I.

In a further embodiment the reaction takes place under inert atmosphere, such as an argon or nitrogen atmosphere.

In a still further embodiment the organic solvent is selected from toluene or a polar aprotic solvent, such as acetonitrile or DMF, and mixtures thereof.

In a further embodiment the suitable temperature is between 15 and 25 °C, such as about room temperature.

In a still further embodiment the temperature is raised in the reaction mixture heating the mixture to 45 °C to 70 °C, such as about 60 °C.

In a further embodiment the reaction is continued for at least 2 hours, such as 3 hours, e.g. from 2.5 to 4 hours.

In a still further embodiment the catalyst is a metal catalyst, such as a metal halide, e.g. Cu(I) or Cu(II), in particular Cu halide, such as Cu iodide.

In a further embodiment the base is present. Typically, the base is an organic base, such as triethylamine or DIPEA.

In a still further embodiment the basic fluoride source agent is TBAF.

In a still further embodiment the molar ratio between the compound of formula IX and trimethyl((3,4,5-trifluorophenyl)ethynyl)silane is 5:4 to 1:3, such as 1:1 to 5:7, typically 5:6, and the organic solvent is in surplus.

In a further embodiment the molar ratio between the compound of formula IX and the catalyst is 20:1 to 2:1, such as 20:1 to 5:1, typically 10:1 and the organic solvent is in surplus.

In a still further embodiment the molar ratio between the compound of formula IX and the base is 1:1 to 1:10, such as 2:3 to 1:3, typically 1:2 and the organic solvent is in surplus.

In a further embodiment the removing of protecting groups in step b) is done by mixing the compound of formula X in an alcohol and with a base in a concentration sufficient to provide a pH of 12 or higher, optionally under inert atmosphere and reacting for at least 15 minutes at a suitable temperature between 15-25 °C, followed by washing with an alkyl ether to obtain the compound of formula I. Typically, the ether is *tert*-Butylmethyl ether (TBME). Preferably, the suitable temperature is 15-25 °C, such as about room temperature. Typically, the organic solvent is selected from an alcohol, such as C₁₋₆ alcohol, e.g. methanol. Furthermore, the base is preferably selected from a base, such as an organic base, in a concentration sufficient to provide a pH of 12 or higher. Typically, the base is sodium methoxide in methanol, such as 25 wt% sodium methoxide solution in methanol.

In an embodiment the reaction with a base is for at least 1 hour, such as 2-24 hours.

In a further embodiment the present process of the invention comprises a step directly preceding step a)
(ia) reacting a compound of formula VIII 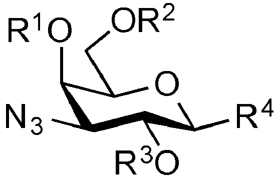 wherein R1, R2, and R3 are independently selected from protecting groups or hydrogen, provided that at least one of R1, R2 and R3 is a protecting group, and R4 is a halogen, with 5-bromopyridine-3-thiol and a base in a suitable organic solvent under suitable conditions, optionally under inert atmosphere, to obtain the compound of formula IX wherein R1, R2, and R3 are independently selected from protecting groups or hydrogen, provided that at least one of R1, R2, and R3 is a protecting group. The base may be selected from NaH, KOtBu, KOH or sodium bis(trimethylsilyl)amide or carbonate bases, e.g. K₂CO₃ and/or Cs₂CO₃.

In an embodiment the compound of formula IX is obtained as a solid.

In a further embodiment the deprotonating agent is sodium bis(trimethylsilyl)amide. In a still further embodiment R1, R2, R3 are all acetyl groups and R4 is as defined above. Preferably, R4 is chlorine.

In a further embodiment the reaction takes place under inert atmosphere. Typically, under an argon or nitrogen atmosphere.

In a still further embodiment the organic solvent is selected from the group consisting of ethyl acetate, THF, toluene, DMF and acetonitrile, and mixtures thereof.

In a further embodiment the suitable conditions in step (ia) are reacting a compound of formula VIII wherein R1, R2, R3 are all acetyl groups and R4 is a halogen, optionally under inert atmosphere and at a suitable temperature below 25 °C with 5-bromopyridine-3-thiol and a base, such as a base selected form NaH, KOtBu, KOH, sodium bis(trimethylsilyl)amide, and/or carbonate bases, e.g. K₂CO₃ and/or Cs₂CO₃, in an organic solvent selected from ethyl acetate, THF, toluene, DMF and acetonitrile, and mixtures thereof, and maintaining the reaction mixture at the suitable temperature, then continue the reaction for at least 15 minutes, and optionally isolating and purifying to obtain the compound of formula IX as a solid. Preferably, the base is cooled to below room temperature before adding 5-bromopyridine-3-thiol over a suitable time and at a suitable temperature and followed by addition of the compound of formula VIII.

In a still further embodiment the suitable temperature is below 25 °C.

In a further embodiment the reaction is continued for at least 2 hours, such as 16-72 hours, at the suitable temperature.

In a still further embodiment the molar ratio between the compound of formula VIII and the base is 1:1 to 1:3, such as 5:7.

In a still further embodiment the process of the present invention comprises a step directly preceding step ia)
(ib) reacting a compound of formula VII 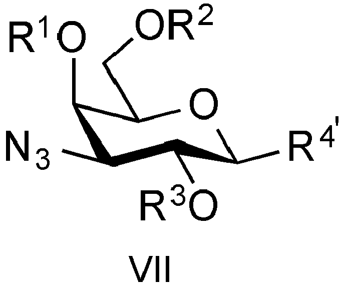 wherein R1, R2, and R3 are independently selected from protecting groups or hydrogen, provided that at least one of R1, R2 and R3 is a protecting group, and R4' is a SR5 or OR5 wherein R5 is selected from H, Z"-C₁₋₆ alkyl, Z"-C₁₋₆ alkenyl, Z"-C₃₋₆ branched alkyl, Z"-C₃₋₆ cyclo alkyl Z"-heteroaryl and Z"-aryl wherein Z" is SO, SO₂, C=O or C=S, with a reagent for activating the anomeric position for nucleophilic substitution, such as a halogenating agent, in a suitable organic solvent, with a suitable catalyst, optionally under inert atmosphere, under suitable conditions to obtain the compound of formula VIII wherein R1, R2, and R3 are independently selected from protecting groups or hydrogen, provided that at least one of R1, R2, and R3 is a protecting group and R4 is a halogen.

In an embodiment R1, R2, and R3 are acetyl groups or hydrogen, provided that at least one of R1, R2 and R3 is an acetyl group, and R4' is OR5 wherein R5 is selected from Z"-C₁₋₆ alkyl wherein Z" is C=O, with a halogenating agent, in an aprotic solvent, with an acid catalyst, optionally under inert atmosphere, at a temperature of between 15 and 45 °C for at least 15 minutes to obtain the compound of formula VIII wherein R1, R2, and R3 are independently selected from acetyl groups or hydrogen, provided that at least one of R1, R2, and R3 is an acetyl group and R4 is a Cl or Br.

In an embodiment the reaction takes place under an inert atmosphere, such as an argon or nitrogen atmosphere.

In a further embodiment R4 is a Cl or Br, such as Cl.
In a further embodiment the organic solvent is an aprotic solvent, preferably dichloromethane, toluene or α,α,α-trifluorotoluene, and mixtures thereof.

In a still further embodiment the reagent for activating the anomeric position for nucleophilic substitution is a halogenating agent. Typically, the halogenating agent is a metal halide, for example, AlCl₃, or a halogenating agent such as SOCl₂, dichloromethyl methyl ether (DCMME) or a halide of phosphorus. Preferably the halogenating agent is PCl₅.

In a further embodiment the catalyst is an acid, such as a lewis acid, preferably BF₃·OEt₂.

In a still further embodiment the suitable conditions involve a suitable temperature of between 15 and 45 °C. In a further embodiment the reaction is continued for at least 15 minutes, at least ½ hour, such as 1-96 hours, at the suitable temperature.

In a still further embodiment the molar ratio between the compound of formula VII and the reagent for activating the anomeric position for nucleophilic substitution such as the halogenating agent, is 5:1 to 1:5, typically 5:6.

In a further embodiment the molar ratio between the compound of formula VII and the catalyst is 10:1 to 200:1, typically 100:1.

In a further aspect the present invention relates to a process of preparing a compound of formula III as well as formula IV starting from compound of formula II.

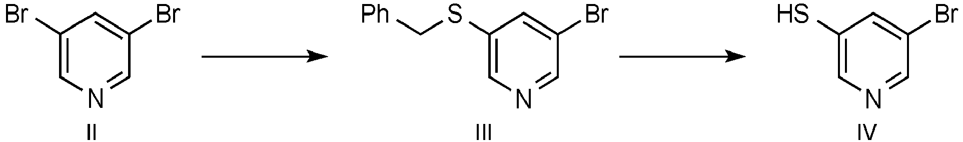

A further aspect concerns a process for preparing a compound of formula III comprising a) treating 3,5-dibromopyridine (II) in an organic solvent and in the presence of a basic bromide source agent, such as TBAB, at a suitable temperature, optionally under an inert atmosphere, and b) adding benzyl mercaptan to obtain the compound of formula III.

A further aspect concerns a process for preparing a compound of formula IV comprising a) treating a 5-bromo-3-mercaptobenzylpyridine (III) in an organic solvent with a reducing agent such as AlCl₃ at a suitable temperature to obtain the compound of formula IV.

A still further concerns a process of preparing a compound of formula VI starting from compound of formula V comprising 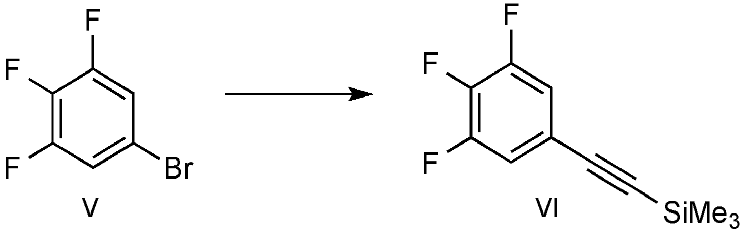
a) treating 5-bromo-1,2,3-trifluorobenzene (V) with a base, such as triethylamine, at a suitable temperature, and optionally a catalyst, such as a metal catalyst, e.g. CuI, and optionally under an inert atmosphere, and b) adding bis(triphenylphosphine) palladium (II) dichloride and ethynyltrimethylsilane in an organic solvent at a suitable temperature to prepare the compound of formula VI.

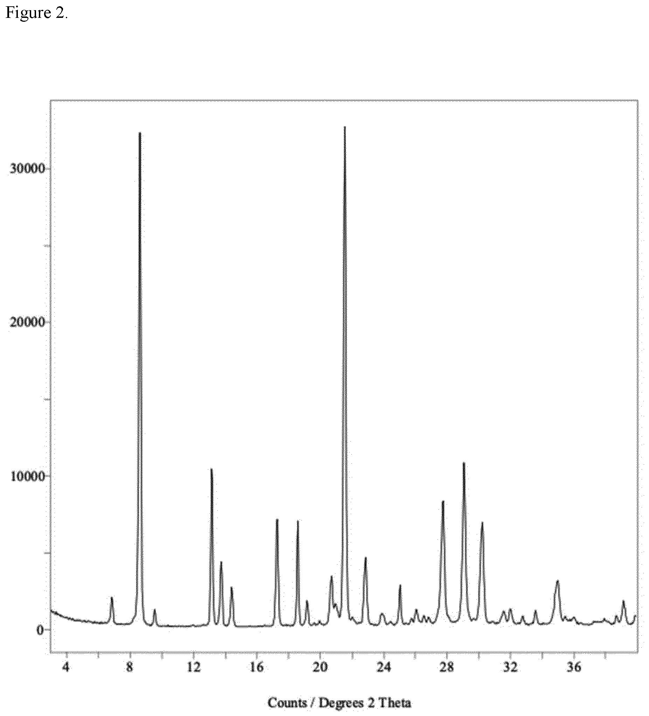

A still further aspect concerns a crystal form of the compound of formula I. In one embodiment the crystalline form is polymorphic form 1 as identified in XRPD diffractogram in Figure 2.

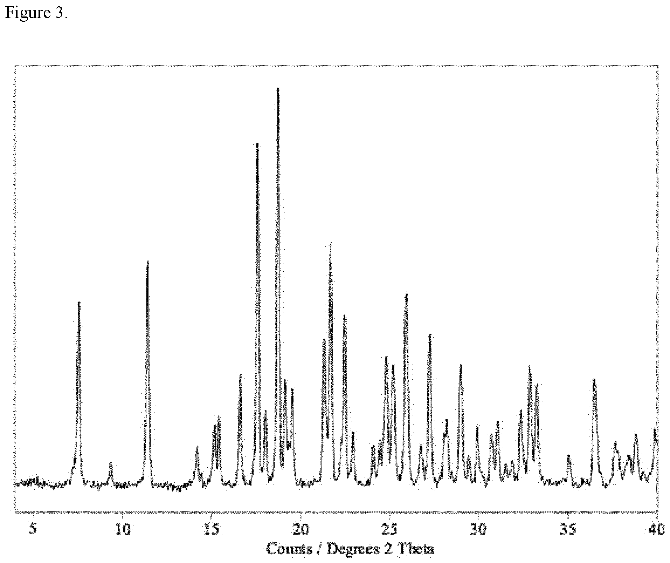

A further aspect concerns a salt of the compound of formula I, preferably the HCl salt of the compound of formula I as identified by the XRPD diffractogram in Figure 3.

The alpha and beta anomers may be separated by various methods such as via crystallization. However, for the present process the starting point can be the mixture as well as one of the anomers.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The disease or disorder to be treated is preferably selected from the group consisting of inflammation; fibrosis, such as pulmonary fibrosis, liver fibrosis, kidney fibrosis, ophthalmological fibrosis and fibrosis of the skin and heart; scarring; keloid formation; aberrant scar formation; surgical adhesions; septic shock; cancer, such as carcinomas, sarcomas, leukemias and lymphomas, such as T-cell lymphomas; metastasising cancers; autoimmune diseases, such as psoriasis, rheumatoid arthritis, Crohn's disease, ulcerative colitis, ankylosing spondylitis, systemic lupus erythematosus; metabolic disorders; heart disease; heart failure; pathological angiogenesis, such as ocular angiogenesis or a disease or condition associated with ocular angiogenesis, e.g. neovascularization related to cancer; and eye diseases, such as age-related macular degeneration and corneal neovascularization; atherosclerosis; metabolic diseases such as diabetes; type 2 diabetes; insulin resistens; obesity; Diastolic HF; asthma and other interstitial lung diseases, including Hermansky-Pudlak syndrome, mesothelioma; liver disorders, such as non-alcoholic steatohepatitis or non-alcoholic fatty liver disease, in a mammal, such as a human, comprising administering a therapeutically effective amount of a composition of the present invention, such as an amorphous solid dispersion composition or a drug layered composition.

Another aspect of the present invention concerns combination therapy involving administering a composition of the present invention, such as an amorphous solid dispersion composition or a drug layered composition, together with a therapeutically active compound different from the compound of formula (I) (interchangeable with "a different therapeutically active compound"). In one embodiment the present invention relates to a combination of a composition of the present invention, such as an amorphous solid dispersion composition or a drug layered composition, and a different therapeutically active compound for use in treatment of a disorder relating to the binding of a galectin-3 to a ligand in a mammal. Such disorders are disclosed below.

In an embodiment of the present invention, a therapeutically effective amount of at least one composition of the present invention, such as an amorphous solid dispersion composition or a drug layered composition, is administered to a mammal in need thereof in combination with a different therapeutically active compound. In a further embodiment, said combination of a composition of the present invention, such as an amorphous solid dispersion composition or a drug layered composition, together with a different therapeutically active compound is administered to a mammal suffering from a disorder selected from the group consisting of inflammation; fibrosis, such as pulmonary fibrosis, liver fibrosis, kidney fibrosis, ophthalmological fibrosis and fibrosis of the skin and heart; scarring; keloid formation; aberrant scar formation; surgical adhesions; septic shock; cancer, such as carcinomas, sarcomas, leukemias and lymphomas, such as T-cell lymphomas; metastasising cancers; autoimmune diseases, such as psoriasis, rheumatoid arthritis, Crohn's disease, ulcerative colitis, ankylosing spondylitis, systemic lupus erythematosus; metabolic disorders; heart disease; heart failure; pathological angiogenesis, such as ocular angiogenesis or a disease or condition associated with ocular angiogenesis, e.g. neovascularization related to cancer; and eye diseases, such as age-related macular degeneration and corneal neovascularization; atherosclerosis; metabolic diseases such as diabetes; type 2 diabetes; insulin resistens; obesity; Diastolic HF; asthma and other interstitial lung diseases, including Hermansky-Pudlak syndrome, mesothelioma; liver disorders, such as non-alcoholic steatohepatitis or non-alcoholic fatty liver disease.

A non-limiting group of cancers given as examples of cancers that may be treated, managed and/or prevented by administration of a composition of the present invention, such as an amorphous solid dispersion composition or a drug layered composition, in combination with a different therapeutically active compound is selected from: colon carcinoma, breast cancer, pancreatic cancer, ovarian cancer, prostate cancer, fibrosarcoma, myxosarcoma, liposarcoma, chondrosarcoma, osteogenic sarcoma, chordoma, angiosarcoma, endotheliosarcoma, lymphangeosarcoma, lymphangeoendothelia sarcoma, synovioma, mesothelioma, Ewing's sarcoma, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystandeocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioblastomas, neuronomas, craniopharingiomas, schwannomas, glioma, astrocytoma, medulloblastoma, craniopharyngioma, ependymoma, pinealoma, hemangioblastoma, acoustic neuroama, oligodendroglioma, meningioma, melanoma, neuroblastoma, retinoblastoma, leukemias and lymphomas, acute lymphocytic leukemia and acute myelocytic polycythemia vera, multiple myeloma, Waldenstrom's macroglobulinemia, and heavy chain disease, acute nonlymphocytic leukemias, chronic lymphocytic leukemia, chronic myelogenous leukemia, Hodgkin's Disease, non-Hodgkin's lymphomas, rectum cancer, urinary cancers, uterine cancers, oral cancers, skin cancers, stomach cancer, brain tumors, liver cancer, laryngeal cancer, esophageal cancer, mammary tumors, childhood-null acute lymphoid leukemia (ALL), thymic ALL, B-cell ALL, acute myeloid leukemia, myelomonocytoid leukemia, acute megakaryocytoid leukemia, Burkitt's lymphoma, acute myeloid leukemia, chronic myeloid leukemia, and T cell leukemia, small and large non-small cell lung carcinoma, acute granulocytic leukemia, germ cell tumors, endometrial cancer, gastric cancer, cancer of the head and neck, chronic lymphoid leukemia, hairy cell leukemia and thyroid cancer.

In some aspects of the present invention, the administration of at least one composition of the present invention, such as an amorphous solid dispersion composition or a drug layered composition, and at least one additional therapeutic agent demonstrates therapeutic synergy. In some aspects of the methods of the present invention, a measurement of response to treatment observed after administering both at least one composition of the present invention, such as an amorphous solid dispersion composition or a drug layered composition, and the additional therapeutic agent is improved over the same measurement of response to treatment observed after administering either the at least one compound of formula (I) of the present invention or the additional therapeutic agent alone.

A further aspect of the present invention concerns combination therapy involving administering a composition of the present invention, such as an amorphous solid dispersion composition or a drug layered composition, together with an anti-fibrotic compound different form the compound of formula (I) to a mammal in need thereof. In a further embodiment, such anti-fibrotic compound may be selected from the following non-limiting group of anti-fibrotic compounds: pirfenidone, nintedanib, simtuzumab (GS-6624, AB0024), BG00011 (STX100), PRM-151, PRM-167, PEG-FGF21, BMS-986020, FG-3019, MN-001, IW001, SAR156597, GSK2126458, PAT-1251 and PBI-4050.

A still further aspect of the present invention concerns combination therapy involving administering a composition of the present invention, such as an amorphous solid dispersion composition or a drug layered composition, in combination with a further conventional cancer treatment such as chemotherapy or radiotherapy, or treatment with immunostimulating substances, gene therapy, treatment with antibodies and treatment using dendritic cells, to a mammal in need thereof.

In an embodiment the composition of the present invention, such as an amorphous solid dispersion composition or a drug layered composition, is administered together with at least one additional therapeutic agent selected from an antineoplastic chemotherapy agent. In a further embodiment, the antineoplastic chemotherapeutic agent is selected from: alltrans retinoic acid, Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Oxaliplatin, Paclitaxel, Pemetrexed, Revlimid, Temozolomide, Teniposide, Thioguanine, Valrubicin, Vinblastine, Vincristine, Vindesine and Vinorelbine. In one embodiment, a chemotherapeutic agent for use in the combination of the present agent may, itself, be a combination of different chemotherapeutic agents. Suitable combinations include FOLFOX and IFL. FOLFOX is a combination which includes 5-fluorouracil (5-FU), leucovorin, and oxaliplatin. IFL treatment includes irinotecan, 5-FU, and leucovorin.

In a further embodiment of the present invention, the further conventional cancer treatment includes radiation therapy. In some embodiments, radiation therapy includes localized radiation therapy delivered to the tumor. In some embodiments, radiation therapy includes total body irradiation.

In other embodiments of the present invention the further cancer treatment is selected from the group of immunostimulating substances e.g. cytokines and antibodies. Such cytokines may be selected from the group consisting of, but not limited to: GM-CSF, type I IFN, interleukin 21, interleukin 2, interleukin 12 and interleukin 15. The antibody is preferably an immunostimulating antibody such as anti-CD40 or anti-CTLA-4 antibodies. The immunostimulatory substance may also be a substance capable of depletion of immune inhibitory cells (e.g. regulatory T-cells) or factors, said substance may for example be E3 ubiquitin ligases. E3 ubiquitin ligases (the HECT, RING and U-box proteins) have emerged as key molecular regulators of immune cell function, and each may be involved in the regulation of immune responses during infection by targeting specific inhibitory molecules for proteolytic destruction. Several HECT and RING E3 proteins have now also been linked to the induction and maintenance of immune self-tolerance: c-Cbl, Cbl-b, GRAIL, Itch and Nedd4 each negatively regulate T cell growth factor production and proliferation.

In some embodiments of the present invention the composition of the present invention, such as an amorphous solid dispersion composition or a drug layered composition, is administered together with at least one additional therapeutic agent selected from a checkpoint inhibitor. In some embodiments of the invention, the checkpoint inhibitor is acting on one or more of the following, non-limiting group of targets: CEACAM1, galectin-9, TIM3, CD80, CTLA4, PD-1, PD-L1, HVEM, BTLA, CD160, VISTA, B7-H4, B7-2, CD155, CD226, TIGIT, CD96, LAG3, GITF, OX40, CD137, CD40, IDO, and TDO. These are known targets and some of these targets are described in Melero et al., Nature Reviews Cancer (2015). Examples of check point inhibitors administered together with the compound of formula (1) are Anti-PD-1: Nivolumab, Pembrolizumab, Cemiplimab. Anti-PD-L1: Atezolizumab, Avelumab, Durvalumab and one Anti-CTLA-4: Ipilimumab. Each one of these check point inhibitors can be made the subject of an embodiment in combination with any one of the compositions of the present invention, such as an amorphous solid dispersion composition or a drug layered composition.

In some embodiments of the present invention the composition of the present invention, such as an amorphous solid dispersion composition or a drug layered composition, is administered together with at least one additional therapeutic agent selected from an inhibitor of indoleamine-2,3-dioxygenase (IDO).

In some embodiments of the present invention the composition of the present invention, such as an amorphous solid dispersion composition or a drug layered composition, is administered together with at least one additional therapeutic agent selected from one or more inhibitors of the CTLA4 pathway. In some embodiments, the inhibitor of the CTLA4 pathway is selected from one or more antibodies against CTLA4.

In some embodiments of the present invention the composition of the present invention, such as an amorphous solid dispersion composition or a drug layered composition, is administered together with at least one additional therapeutic agent selected from one or more inhibitors of the PD-1/PD-L pathway. In some embodiments, the one or more inhibitors of the PD-1/PD-L pathway are selected from one or more antibodies against PD-1, PD-L1, and/or PD-L2.

As used herein "pharmaceutically acceptable additive" is intended without limitation to include carriers, excipients, diluents, adjuvant, colorings, aroma, preservatives etc. that the skilled person would consider using when formulating a compound of the present invention in order to make a pharmaceutical composition.

The adjuvants, diluents, excipients and/or carriers that may be used in the composition of the invention must be pharmaceutically acceptable in the sense of being compatible with the compound of formula (I) and the other ingredients of the pharmaceutical composition, and not deleterious to the recipient thereof. It is preferred that the compositions shall not contain any material that may cause an adverse reaction, such as an allergic reaction. The adjuvants, diluents, excipients and carriers that may be used in the pharmaceutical composition of the invention are well known to a person within the art.

Further embodiments of the process are described in the experimental section herein, and each individual process as well as each starting material constitutes embodiments that may form part of embodiments.

The above embodiments should be seen as referring to any one of the aspects (such as 'method for treatment', 'pharmaceutical composition', 'compound for use as a medicament', or 'compound for use in a method') described herein as well as any one of the embodiments described herein unless it is specified that an embodiment relates to a certain aspect or aspects of the present invention.

All references, including publications, patent applications and patents, cited herein are hereby incorporated by reference to the same extent as if each reference was individually and specifically indicated to be incorporated by reference and was set forth in its entirety herein.

All headings and sub-headings are used herein for convenience only and should not be construed as limiting the invention in any way.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (*e.g*., all exact exemplary values provided with respect to a particular factor or measurement can be considered to also provide a corresponding approximate measurement, modified by "about," where appropriate).

All methods described herein can be performed in any suitable order unless otherwise indicated herein or otherwise clearly contradicted by context.

The use of any and all examples, or exemplary language (*e.g*., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of', "consists essentially of', or "substantially comprises" that particular element or elements, unless otherwise stated or clearly contradicted by context *(e.g.,* a composition described herein as comprising a particular element should be understood as also describing a composition consisting of that element, unless otherwise stated or clearly contradicted by context).

This invention includes all modifications and equivalents of the subject matter recited in the aspects or claims presented herein to the maximum extent permitted by applicable law.

The present invention is further illustrated by the following examples that, however, are not to be construed as limiting the scope of protection. The features disclosed in the foregoing description and in the following examples may, both separately and in any combination thereof, be material for realizing the invention in diverse forms thereof.

### Experimental

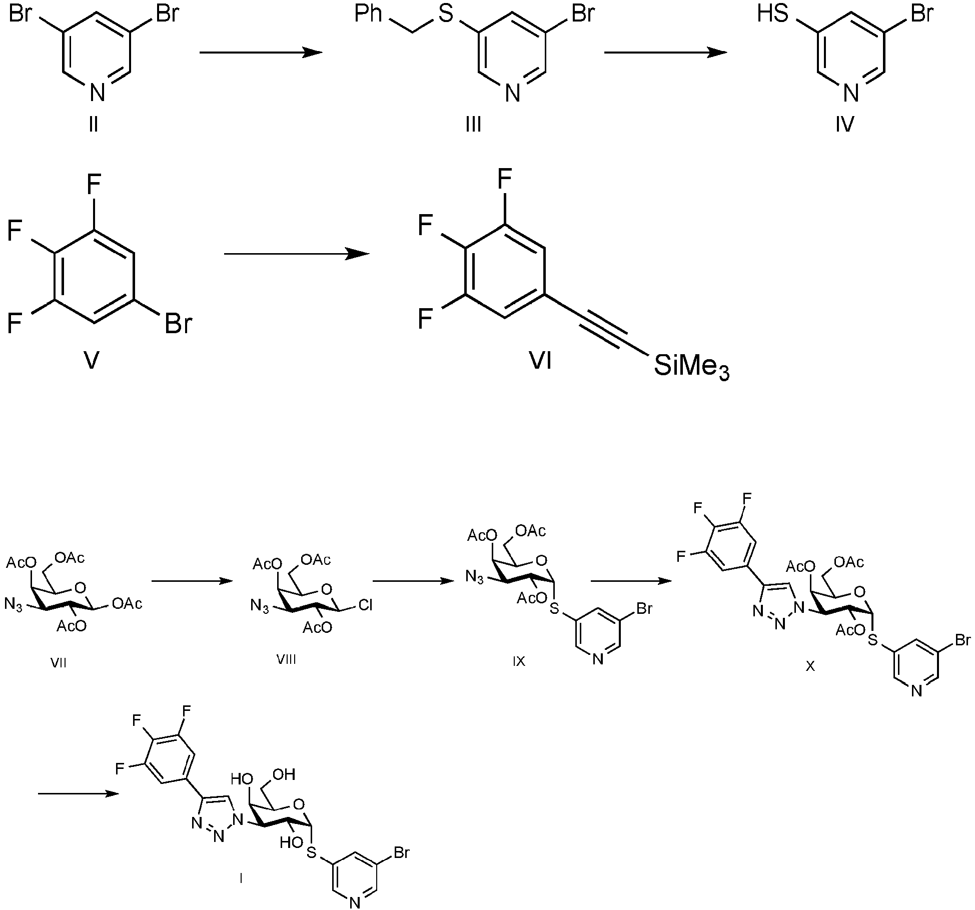

The current process to manufacture 5-Bromopyridin-3-yl 3-deoxy-3-[4-(3,4,5-trifluorophenyl)-1*H*-1,2,3-triazol-1-yl]-1-thio-α-D-galactopyranoside having formula I involves several process steps as described in detail hereunder.

### General Procedures

Nuclear Magnetic Resonance (NMR) spectra were recorded on a 400 MHz Bruker Avance AV400 spectrometer at 25 °C. Chemical shifts are reported in ppm (δ) using the residual solvent as the internal standard. Peak multiplicities are expressed as follows: s, singlet; d, doublet; t, triplet; q, quartet; m, multiplet; br s, broad singlet.

The following abbreviations are used:
- Ac:: Acetyl
- aq.:: aqueous
- DCM:: Dichloromethane
- DMF:: *N,N*-Dimethylformamide
- DMSO:: Dimethylsulfoxide
- Sat.:: Saturated
- TBAB:: Tetra-n-butylammonium bromide
- TBAF:: Tetra-n-butylammonium fluoride
- TBME: *tert*-Butylmethyl ether
- TLC:: thin layer chromatography

### 5-Bromopyridine-3-thiol

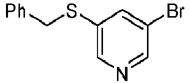

To a jacketed vessel fitted with an aq. NaOCl filled scrubber was charged 3,5-dibromopyridine (5 kg, 21.1 mol), TBAB (308 g, 0.95 mol) and toluene (11.9 L) and the mixture was stirred under argon. 50% aq. NaOH (11.9 L) was added and the mixture heated to 30 °C. Benzyl mercaptan (2.25 L, 19.1 mol) was added over 2 h maintaining the temperature 30 °C ± 3 °C. The mixture was stirred for an additional 30 minutes then cooled to 20 °C. The aqueous phase was removed and the organic phase was washed with 10% NaCl solution (3 x 11.9 L). The organic phase was dried over MgSO₄, filtered to give a solution of 5-bromo-3-mercaptobenzylpyridine which was used directly in the next step.

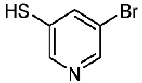

To a jacketed vessel was charged AlCl₃ (4.35 kg, 32.6 mol) and toluene (16.1 L) and the mixture was stirred and cooled to -5 °C. The solution of 5-bromo-3-mercaptobenzylpyridine (19.1 mol) was added over 2-3 h maintaining the temperature below 5 °C. The resulting mixture was then quenched by the addition of water (16.1 L) over 3 h maintaining the temperature below 20 °C. The phases were separated and the organic phase was washed with water (2 x 16.1 L) then extracted with 10% aq. NaOH solution (2 x 2.68 L). The combined aqueous phases were washed with toluene (2 x 5.37 L), then to the aqueous phase at 5 °C under argon sparging was added concentrated HCl until pH 2.5 was attained. The mixture was extracted with ethyl acetate (3 x 5.37 L) and the combined organic phases were subsequently washed with 10% aq. NaCl solution (2 x 5.37 L), dried over MgSO₄ and concentrated in vacuo to give 2.99 kg (82%) of 5-bromopyridine-3-thiol as an orange solid. ¹H NMR (400 MHz, CDCl₃) δ 8.45 (d, *J* = 2.0 Hz, 1H), 8.41 (d, *J* = 2.0 Hz, 1H), 7.77 (t, *J* =2.0 Hz, 1H).

### Trimethyl((3,4,5-trifluorophenyl)ethynyl)silane

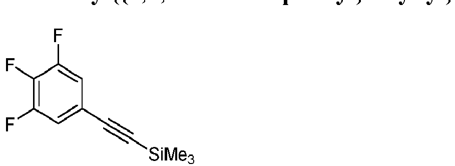

To a jacketed vessel was charged triethylamine (5.45 L, 39.1 mol), 5-bromo-1,2,3-trifluorobenzene (2.75 kg, 13.0 mol) and CuI (191 g, 0.65 mol) and the mixture was heated to reflux under argon. A solution of bis(triphenylphosphine) palladium (II) dichloride (91.5 g, 0.13 mol) and ethynyltrimethylsilane (2.40 L, 17.3 mol) in DMF (27.5 L) was charged to the vessel over 2 h. The mixture was stirred for 3 h then cooled to 20 °C and filtered. The filtrate was re-charged to the vessel and diluted with TBME (13.75 L) and 2M aq. HCl solution (13.75 L) was added maintaining the temperature less than 30 °C. The aqueous phase was drained and the organic phase was subsequently washed with 6 % aq. NH₄OH solution (3 x 13.75 L) and 10% aq. NaCl solution (13.75 L). The organic phase was concentrated in vacuo at 40 °C and the filtrate was slurried in heptane (13.75 L) for 1 h at 20 °C and filtered through a bed of celite. The filtrate was concentrated in vacuo at 40 °C to give 2.11 kg (71%) of trimethyl((3,4,5-trifluorophenyl)ethynyl)silane as a brown oil. ¹H NMR (400 MHz, CDCl₃) δ 7.11-7.01 (m, 2H), 0.24 (s, 9H). ¹⁹F NMR (376 MHz, CDCl₃) δ -134.3 (d, *J* = 20.5 Hz, 2F), -158.6 (t, *J* = 20.5 Hz, IF).

### Alternative procedure:

To a jacketed vessel was charged triethylamine (1.39 L, 9.95 mol), 5-bromo-1,2,3-trifluorobenzene (700 g, 3.32 mol), CuI (31.6 g, 0.17 mol), bis(triphenylphosphine) palladium (II) dichloride (58.2 g, 0.08 mol) and acetonitrile (2.1 L). The mixture was heated to reflux under argon. A solution of ethynyltrimethylsilane (611 mL, 4.41 mol) in acetonitrile (4.9 L) was charged to the vessel over 2 h. The mixture was stirred for 2 h at reflux then cooled to 25 °C and filtered through a bed of celite. The filtrate was concentrated in vacuo at 40 °C. MP-TMT resin (145.5 g) was added to the crude. The mixture was slurried in 9:1 heptane:ethyl acetate (7 L) for 2 h at 30 °C and filtered through a bed of celite. The filter cake was washed with 9:1 heptane:ethyl acetate (7 L) and the filtrate was concentrated in vacuo at 40 °C to give 698 g (93%) of trimethyl((3,4,5-trifluorophenyl)ethynyl)silane as a brown oil.

### 2,4,6-Tri-O-acetyl-3-azido-3-deoxy-β-D-galactopyranosyl chloride

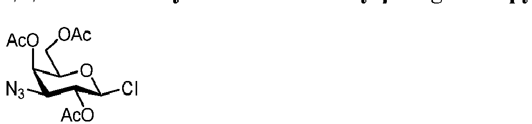

To a solution of PCl₅ (335 g, 1.61 mol) in DCM (1.5 L) under argon was added BF₃·OEt₂ (1.65 mL, 0.013 mol) followed by a solution of 1,2,4,6-Tetra-*O*-acetyl-3-azido-3-deoxy-β-D-galactopyranoside (500 g, 1.34 mol) in DCM (1 L), maintaining the temperature less than 25 °C. The reaction mixture was stirred in the range 20 °C ± 5 °C for 1 hour when complete consumption of starting material was observed by TLC. The mixture was cooled to 5 °C and a solution of 20% aq. KHCO₃ (2 L) was added over 20 minutes maintaining the temperature less than 20 °C. The mixture was stirred for 15 minutes and then the layers were separated. To the stirred organic phase was added a further charge of 20% aq. KHCO₃ solution (2 L) was added over 10 minutes maintaining the temperature less than 20 °C. The mixture was stirred for 15 minutes and then the layers were separated. The organic phase was dried over MgSO₄ (250 g), filtered and concentrated in vacuo at 40 °C to give an off-white solid. The crude material was slurried on a rotary evaporator with TBME (1 L) for 1 h at 40 °C at atmospheric pressure then cooled to 5 °C and held for 16 h. The material was collected by filtration, the filter cake washed with TBME (80 mL) and the material dried by pulling air through the filter to yield 330 g (70%) of 2,4,6-Tri-*O*-acetyl-3-azido-3-deoxy-β-D-galactopyranosyl chloride as an off-white solid. ¹H NMR (400 MHz, CDCl₃) δ 5.41 (dd, *J* = 3.1, 1.1 Hz, 1H), 5.27 (dd, *J =* 10.2, 8.8 Hz, 1H), 5.17 (d, *J* = 8.8 Hz, 1H), 4.11 (dd, *J* = 11.6, 6.1 Hz, 1H), 4.03 (dd, *J* = 11.6, 6.6 Hz, 1H), 3.91 (td, *J* = 6.6, 1.2 Hz.1H), 3.54 (dd, *J* = 10.2, 3.3 Hz, 1H), 2.13 (s, 3H), 2.10 (s, 3H), 2.00 (s, 3H).

### Alternative procedure:

PCl₅ (1.00 kg, 4.8 mol) was charged to a jacketed vessel under argon. A solution of BF₃·OEt₂ (5 mL, 0.04 mol) in α,α,α-trifluorotoluene (3 L) was charged and the mixture was heated to 40 °C. A solution of 1,2,4,6-Tetra-*O*-acetyl-3-azido-3-deoxy-β-D-galactopyranoside (1.50 kg, 4.02 mol) in α,α,α-trifluorotoluene (6.75 L) was dosed to the reaction mixture, maintaining the temperature 35 °C ± 5 °C. A line rinse was conducted with α,α,α-trifluorotoluene (0.75 L). The reaction mixture was stirred in the range of 35 °C ± 5 °C for 1 hour. The mixture was cooled to -5 °C, cyclohexane (4.5 L) was charged over 30 minutes, and the resulting suspension was stirred for 16 h. The reaction mixture was filtered under argon. The filter cake was dried in a vacuum oven at 20 °C for 3 hours to yield 942 g (67 %) of 2,4,6-Tri-*O*-acetyl-3-azido-3-deoxy-β-D-galactopyranosyl chloride as an off-white solid.

### 5-Bromopyridin-3-yl 2,4,6-tri-O-acetyl-3-azido-3-deoxy-1-thio-α-D-galactopyranoside

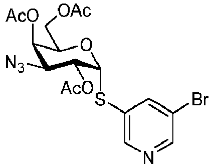

A jacketed vessel was charged with sodium bis(trimethylsilyl)amide solution (4.2 L, 2 M solution in THF, 8.37 mol) and the solution was stirred at 5 °C under argon. A solution of 5-bromopyridine-3-thiol (1.6 kg, 8.37 mol) in THF (2.1 L) was added over 1 h 15 min maintaining the temperature less than 20 °C. To the mixture was subsequently added a solution of 2,4,6-Tri-*O*-acetyl-3-azido-3-deoxy-β-D-galactopyranosyl chloride (2.1 kg, 5.98 mol) in THF (2.1 L) over 15 minutes. A line rinse was conducted with additional THF (1 L). The resulting mixture was stirred for 18 h then TBME (6.3 L) was charged and the temperature of the mixture reduced to 10 °C. Water (6.3 L) was added over 20 minutes and the resulting mixture was stirred for 30 minutes. The phases were separated and the aqueous phase was extracted with TBME (6.3 L). The combined organic phases were washed with 10% aq. NaCl solution (3 x 6.3 L) then concentrated in vacuo at 40 °C. The crude material was co-evaporated in vacuo with methanol (4.2 L) then slurried in methanol (4.2 L) at 50 °C for 2 h. The mixture was cooled to 20 °C then filtered and the filter cake washed with methanol (1 L). The solid material was further dried to a constant mass in vacuo at 40 °C to give 2.09 kg (69%) of 5-Bromopyridin-3-yl 2,4,6-tri-*O*-acetyl-3-azido-3-deoxy-1-thio-α-D-galactopyranoside. ¹H NMR (400 MHz, CDCl₃) δ 8.57 (dd, *J* = 10.5, 2.2 Hz, 2H), 7.96 (t, *J* = 2.1 Hz, 1H), 5.98 (d, *J* = 5.5 Hz, 1H), 5.52-5.45 (m, 1H), 5.27 (dd, *J* = 11.0, 5.5 Hz, 1H), 4.63 (ddd, *J* = 7.8, 4.6, 0.8 Hz, 1H), 4.13 (dd, *J* 11.7, 4.3 Hz, 1H), 4.02 (dd, *J* 11.7, 7.8 Hz, 1H), 3.96 (dd, *J* 11.0, 3.3 Hz, 1H), 2.20 (s, 3H), 2.17 (s, 3H). 2.03 (s, 3H).

### Alternative procedure:

A jacketed vessel was charged with sodium bis(trimethylsilyl)amide solution (2.4 L, 2 M solution in THF, 4.80 mol) and the solution was stirred at 5 °C under argon. A solution of 5-bromopyridine-3-thiol (913 g, 4.80 mol) in THF (1.2 L) was added over 1 h 15 min maintaining the temperature less than 20 °C. A line rinse was conducted with additional THF (0.6 L). To the mixture was subsequently added a solution of 2,4,6-Tri-*O*-acetyl-3-azido-3-deoxy-β-D-galactopyranosyl chloride (1.2 kg, 3.43 mol) in THF (1.2 L) over 15 minutes. A line rinse was conducted with additional THF (0.6 L). The resulting mixture was stirred for 72 hours at 20 °C. The temperature of the mixture was reduced to 5 °C and water (3.6 L) was added over 45 minutes followed by ethyl acetate (3.6 L). The resulting mixture was stirred for 1 hour at 20 °C. The phases were separated and the aqueous phase was extracted with ethyl acetate (3.6 L). The combined organic phases were washed with 10% aq. NaCl solution (3 x 3.6 L) then concentrated in vacuo at 40 °C. The crude material was co-evaporated in vacuo with methanol (2.4 L) then slurried in methanol (2.4 L) at 50 °C for 2 hours. The mixture was cooled to 20 °C then filtered and the filter cake washed with methanol (0.5 L). The solid material was dried to a constant mass in vacuo at 40 °C to give 1.21 kg (70%) of 5-Bromopyridin-3-yl 2,4,6-tri-*O*-acetyl-3-azido-3-deoxy-1-thio-α-D-galactopyranoside.

### 5-Bromopyridin-3-yl 2,4,6-tri-O-acetyl-3-deoxy-3-[4-(3,4,5-trifluorophenyl)-1H-1,2,3-triazol-1-yl]-1-thio-α-D-galactopyranoside

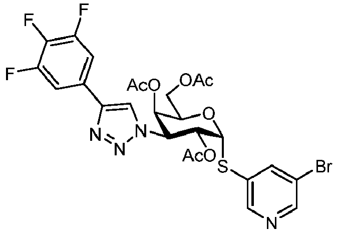

A jacketed vessel was charged with trimethyl((3,4,5-trifluorophenyl)ethynyl)silane (1.11 kg, 4.88 mol), CuI (81.68 g, 0.41 mol) and acetonitrile (20 L) and the mixture was stirred under argon. 5-Bromopyridin-3-yl 2,4,6-tri-*O*-acetyl-3-azido-3-deoxy-1-thio-α-D-galactopyranoside (2.02 kg) and triethylamine (1.2 L, 8.15 mol) were charged and the mixture was heated to 60 °C. Three portions of 1M TBAF solution in THF (3 x 200 mL, 0.20 mol) were added every 30 minutes and the reaction was stirred for a further 30 minutes after the final addition. Ethyl acetate (20 L) was charged and the mixture was cooled to 20 °C. The mixture was washed with 10% NH₄OH solution (4 x 6 L) until the aqueous phase no longer turns blue. The organic phase was subsequently washed with 2M aq. HCl solution (2 x 6 L), 5% aq. NaHCO₃ solution (6 L) and then concentrated in vacuo at 50 °C. The 5-Bromopyridin-3-yl 2,4,6-tri-*O*-acetyl-3-deoxy-3-[4-(3,4,5-trifluorophenyl)-1*H*-1,2,3-triazol-1-yl]-1-thio-α-D-galactopyranoside was used in the next step without further purification. ¹H NMR (400 MHz, CDCl₃) δ 8.61 (dd, *J* = 6.3, 2.0 Hz, 2H), 8.00 (t, *J* = 2.0 Hz, 1H), 7.79 (s, 1H), 7.48-7.39 (m, 2H). 6.14 (d, *J* = 5.7 Hz, 1H), 6.09 (dd, *J* = 11.5, 5.5 Hz, 1H), 5.62 (dd, *J* = 3.0, 1.1 Hz, 1H), 5.22 (dd, *J* = 11.4, 3.1 Hz, 1H), 4.89-4.81 (m, 1H), 4.16 (dd, *J* = 11.7, 4.9 Hz, 1H), 4.08 (dd, *J* = 11.7, 7.6 Hz, 1H), 2.06 (s, 3H), 2.04 (s, 3H), 1.98 (s, 3H). Evidence of the formation of 5-Bromopyridin-3-yl 4,6-di-*O*-acetyl-3-deoxy-3-[4-(3,4,5-trifluorophenyl)-1*H*-1,2,3-triazol-1-yl]-1-thio-α-D-galactopyranoside was also observed by ¹H NMR spectroscopy. ¹H NMR (400 MHz, CDCl₃) δ 8.60 (dd, *J* = 14.0, 2.0, 2H), 8.07 (t, *J* = 2.0, 1H), 7.90 (s, 1H), 7.42-7.32 (m, 2H), 5.93 (d, *J* = 5.3 Hz, 1H), 5.64 (dd, *J* = 3.0, 1.1, 1H), 5.27 (dt, *J* = 10.9, 5.6, 1H), 4.93 (dd, *J* = 10.9, 3.0 Hz, 1H), 4.87-4.79 (m, 1H), 4.43 (d, *J* = 5.8 Hz, 1H), 4.21-4.05 (m, 2H), 2.04 (s, 3H), 2.01 (s, 3H).

### 5-Bromopyridin-3-yl 3-deoxy-3-[4-(3,4,5-trifluorophenyl)-1H-1,2,3-triazol-1-yl]-1-thio-α-D-galactopyranoside, I

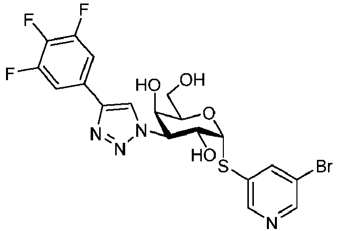

The crude 5-Bromopyridin-3-yl 2,4,6-tri-*O*-acetyl-3-deoxy-3-[4-(3,4,5-trifluorophenyl)-1*H-*1,2,3-triazol-1-yl]-1-thio-α-D-galactopyranoside (5.36 kg, 8.13 mol) was dissolved in methanol (13.4 L) and stirred under argon. 25 wt% sodium methoxide solution (185 mL, 0.81 mol) was charged and the mixture was stirred at 20 °C for 16 h. Additional 25 wt% sodium methoxide solution (90 mL, 0.41 mol) was charged and the mixture was stirred at 20 °C for 24 h. TBME (8.5 L) was charged and the mixture stirred for 2 h at 20 °C. The mixture was filtered and the filter cake was washed with TBME (5.3 L). The solid material was dried to a constant mass in vacuo at 30 °C to give 2.79 kg of 5-Bromopyridin-3-yl 3-deoxy-3-[4-(3,4,5-trifluorophenyl)-1*H*-1,2,3-triazol-1-yl]-1-thio-α-D-galactopyranoside as an off-white solid (65% over 2 steps). ¹H NMR (400 MHz, DMSO-d₆) δ 8.81 (s, 1H), 8.68 (d, *J* = 1.9 Hz, 1H), 8.61 (d, *J* = 2.1 Hz, 1H), 8.30 (t, *J* = 2.1 Hz, 1H), 7.90-7.80 (m, 2H), 5.99 (d, *J* = 5.2 Hz, 1H), 5.96 (br s, 1H), 5.54 (d, *J* = 5.6 Hz, 1H), 4.85 (dd, *J* = 11.3, 2.8 Hz, 1H), 4.81-4.69 (m, 2H), 4.27 (t, *J* = 6.2 Hz, 1H), 4.08-4.00 (m, 1H), 3.59-3.49 (m, 1H), 3.46-3.36 (m, 1H).

### Polymorphs and Salt form preparation:

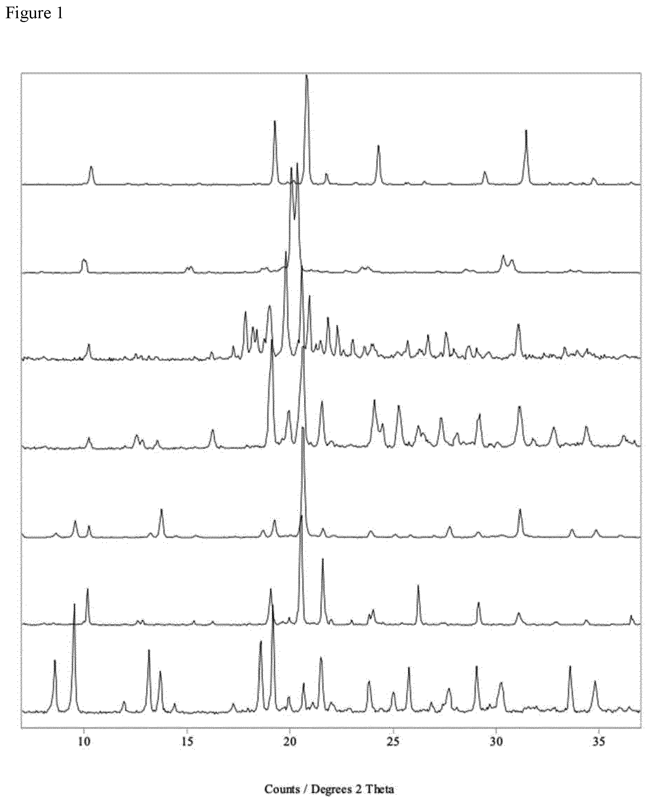

5-Bromopyridin-3-yl 3-deoxy-3-[4-(3,4,5-trifluorophenyl)-1H-1,2,3-triazol-1-yl]-1-thio-α-D-galactopyranoside is a crystalline solid that can potentially exist as 7 crystalline polymorphs as well as an amorphous form. Five of the seven solid forms are solvated forms (Forms 2, 4, 5, 6, and 7), crystallize from an alcohol solvent, and appear to be unique but structurally related (pseudo-isostructural) solvates. Table 1 lists the polymorphic forms and the key solvents they are generated from. Figure 1 provides the XRPD diffractograms for the polymorphic forms identified.

**Table 1: Polymorph Screen of Compound I**

| Form Designation | Description | Key Solvents |
|---|---|---|
| Form 1 | Stable non-solvated polymorph | multiple |
| Form 2 | Solvate | ethanol, isopropanol |
| Form 3 | Possible solvate or polymorph | multiple |
| Form 4 | Isolated form from alcohol slurry | ethanol, isopropanol |
| Form 5 | solvate | isopropanol, isobutanol |
| Form 6 | Suspected solvate | isobutanol |
| Form 7 | solvate | 1:1 TFE:acetone |

In figure 1 the XRPD patterns for polymorphic forms of the Compound of formula I can be identified: From bottom to top: Form 1, Form 2, Form 3, Form 4, Form 5, Form 6, Form 7.

The compound of formula (I) is designated polymorphic Form 1 as identified in XRPD diffractogram in Figure 2. The polymorphic Form 1 of the compound of formula (I) is a highly crystalline form with a melting point of 233.7°C. Form 1 is not hygroscopic and shows no indication of hydrate or solvate formation.

The polymorphic forms of the Compound of formula I can be prepared by the process comprising the steps of suspending or dissolving 3,3'-Dideoxy-3,3'-bis-[4-(3-fluorophenyl)-1H-1,2,3-triazol-1-yl]-1,1'-sulfanediyl-di-β-D-galactopyranoside in an organic solvent or mixture of solvents (see table 2 below) and then using fast evaporation, slow evaporation, equilibrated slurry and precipitation from solvent by adding anti-solvent, or a combination thereof to prepare the polymorphs. Samples by fast and slow evaporation were generated by mixing 7 mg of 3,3'-Dideoxy-3,3'-bis-[4-(3-fluorophenyl)-1H-1,2,3-triazol-1-yl]-1,1'-sulfanediyl-di-β-D-galactopyranoside with a solvent (see Table 2 below) and sonicating to ensure complete dissolution. For fast evaporation, a genovac centrifugal evaporator was used to remove low boiling solvents over 20 minutes at controlled vacuum. For slow evaporation, the solvents were allowed to evaporate over 24 hours. Samples subjected to equilibrium slurry conditions were prepared by mixing 7 mg of 3,3'-Dideoxy-3,3'-bis-[4-(3-fluorophenyl)-1H-1,2,3-triazol-1-yl]-1,1'-sulfanediyl-di-β-D-galactopyranoside with a solvent (see Table 2 below). The resulting slurries were sonicated in a bath followed by stirring for 48 hours. Solids were isolated by filtration onto sintered filters. Samples subjected to precipitation with anti-solvent were prepared by mixing 7 mg of 3,3'-Dideoxy-3,3'-bis-[4-(3-fluorophenyl)-1H-1,2,3-triazol-1-yl]-1,1'-sulfanediyl-di-β-D-galactopyranoside with a solvent (see Table 2 below) and mixed until dissolution achieved. Anti-solvent (see Table 2 below) was added to rapidly precipitate a solid which was isolated by vacuum filtration onto sintered metal filters. Table 2 lists each form generated by these methods. Form 1 was also made by the process described in the large scale method described above as on off-white solid.

**Table 2: List of Solvents in Polymorph Generation Experiments**

| Solvent | Fast Evaporation | Slow Evaporation | Equilibrium Slurry | Precipitation from Anti-Solvent | |
|---|---|---|---|---|---|
| | Form generated | Form generated | Form generated | Anti-solvent | Form generated |
| Ethyl Acetate | - | Form 1 | Form 1 | - | |
| Ethanol | Form 2 | Form 2 | Form 4 | - | |
| Isopropanol | Form 2 | Form 5 | Form 4 | - | |
| Isobutanol | Form 6 | Form 5 | - | - | |
| Tetrahydrofuran | Form 1 | Form 1 | - | MTBE | Form 1 |
| | | | | Heptane | From 3 |
| | | | | DCM | From 1 |
| Propyl acetate | Form 1 | Form 1 | - | | |
| Methanol | Form 1 | Form 1 | - | MTBE | Form 1 |
| | | | | Heptane | From 2 |
| | | | | DCM | From 1 |
| 1:1 TFE:acetone | Form 3 | Form 7 | - | - | |
| acetonitrile | Form 1 | Form 1 | Form 1 | - | |
| Butyl alcohol | Form 3 | Form 1 | Form 1 | - | |
| acetone | Form 1 | Form 1 | - | MTBE | Form 1 |
| | | | | Heptane | From 3 |
| | | | | DCM | From 1 |
| Methyl ethyl ketone | Form 1 | Form 1 | Form 1 | - | |
| propionitrile | Form 1 | Form 1 | Form 1 | - | |
| 3:1 isopropanol:THF | Form 1 | Form 5 | - | - | |
| 1:1 propyl acetate: THF | Form 1 | Form 1 | - | - | |
| 3:1 water:THF | Form 1 | Form 1 | Form 1 | - | |
| 1:6 water:acetone | Form 1 | Form 1 | - | - | |
| 1:4 water: ethanol | Form 1 | Form 2 | - | - | |
| 1:1 water: methanol | Form 1 | - | - | - | |
| 1:3 water:acetone | - | - | Form 1 | - | |
| 2:1 propyl acetate:THF | - | - | Form 1 | - | |
| 1:2 water:ethanol | - | - | Form 2 | - | |
| water | = | - | Form 1 | - | |

3,3'-Dideoxy-3,3'-bis-[4-(3-fluorophenyl)-1H-1,2,3-triazol-1-yl]-1,1'-sulfanediyl-di-β-D-galactopyranoside can exist in a variety of salt forms including hydrochloride, hydrobromide, sulfate, phosphate, ethane sulfonate and methane sulfonate. Salt forms of the Compound of formula I can be prepared via fast evaporation or slurry conversion of a 1:1 mixture of Compound of formula I and an acid such as sulfuric, hydrochloric, hydrobromic, phosphoric, ethane sulfuric and methane sulfuric acids in an appropriate solvent, such as methyl ethyl ketone, acetonitrile, acetone, ethanol, heptane, ethyl acetate, water or mixtures of the listed solvents. A typical example of a salt form of Compound of formula I is the hydrochloride salt (HCl salt) which is a crystalline salt with a melting point of 221°C. The HCl salt is identified by the XRPD diffractogram in Figure 3. The HCl salt of the compound of formula I was made by mixing 7 mg of 3,3'-Dideoxy-3,3'-bis-[4-(3-fluorophenyl)-1H-1,2,3-triazol-1-yl]-1,1'-sulfanediyl-di-β-D-galactopyranoside with an equimolar amount of hydrochloric acid and a mixture of 1:1 ethyl acetate:heptane at a concentration of 10 mg/mL in a vial. The vial was sealed, and the mixture stirred for 36 hours. The resulting solids were isolated by filtration onto sintered metal filters.

## Claims

1. A process suitable for large scale synthesis for preparing 5-Bromopyridin-3-yl 3-deoxy-3-[4-(3,4,5-trifluorophenyl)-1H-1,2,3-triazol-1-yl]-1-thio-α-D-galactopyranoside having formula (I) 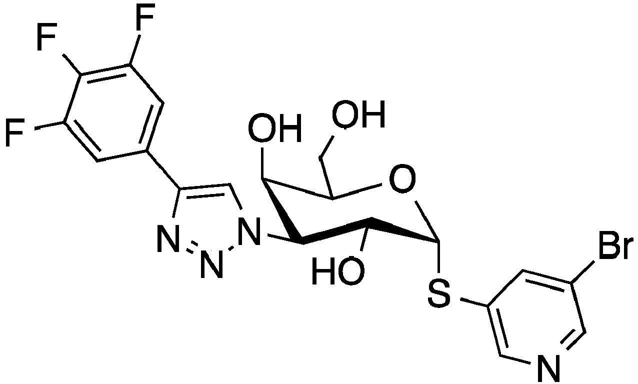 wherein the process comprises the consecutive steps of
a) reacting a compound of formula IX 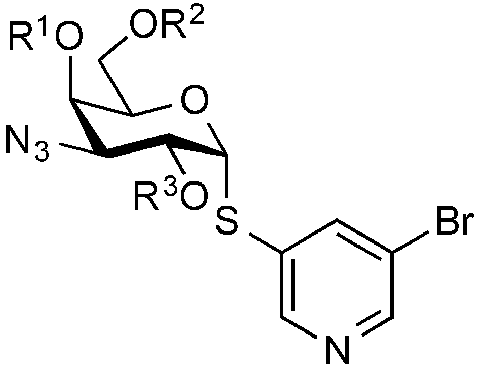 wherein R1, R2, and R3 are independently selected from protecting groups or hydrogen, provided that at least one of R1, R2, and R3 is a protecting group, with 5-ethynyl-1,2,3-trifluorobenzene) or a silane protected 5-ethynyl-1,2,3-trifluorobenzene), such as trimethyl((3,4,5-trifluorophenyl)ethynyl)silane, and a catalyst and optionally adding a base in an organic solvent, and optionally adding a basic fluoride source agent, such as TBAF, under suitable conditions to obtain a compound of formula X 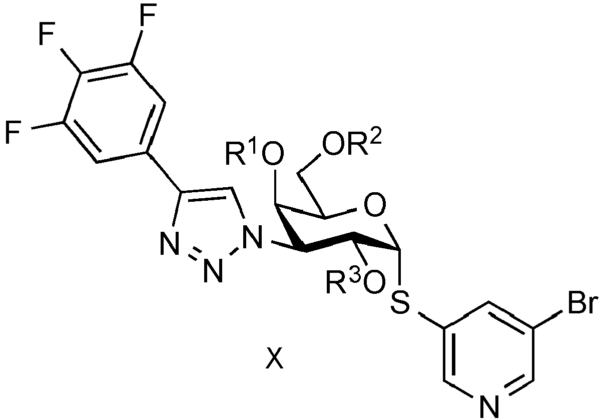 wherein R1, R2, R3 are as defined above, and
b) removing the protecting groups of the compound of formula X to obtain the compound of formula I.

2. The process of claim 1 wherein the suitable conditions in step a) are reacting a compound of formula IX wherein R1, R2, R3 are independently selected from protecting groups or hydrogen, provided that at least one of R1, R2, R3, is a protecting group, with trimethyl((3,4,5-trifluorophenyl)ethynyl)silane in the organic solvent at a suitable temperature, optionally under inert atmosphere, and adding a catalyst and optionally a base in the organic solvent to create a reaction mixture and optionally heating the reaction mixture to raise the temperature at least 15 °C above the suitable temperature, and adding the basic fluoride source agent and continue the reaction for at least 1 hour to obtain the compound of formula X wherein R1, R2, R3 are as defined above.

3. The process of any one of claims 1-2 wherein R1, R2, R3 are independently selected form ester protecting groups.

4. The process of any one of claims 1-3 wherein the reaction takes place under inert atmosphere.

5. The process of any one of claims 1-4 wherein the organic solvent is selected from toluene or a polar aprotic solvent.

6. The process of any one of claims 1-5 wherein the suitable temperature is between 15 and 25 °C.

7. The process of any one of claims 1-6 wherein the temperature is raised in the reaction mixture heating the mixture to 45 °C to 70 °C.

8. The process of any one of claims 1-7 wherein the reaction is continued for at least 2 hours.

9. The process of any one of claims 1-8 wherein the catalyst is a metal catalyst.

10. The process of any one of claims 1-9 wherein the base is an organic base.

11. The process of any one of claims 1-10 wherein the basic fluoride source agent is TBAF.

12. The process of any one of claims 1-11 wherein the removing of protecting groups in step b) is done by mixing the compound of formula X in an organic solvent and with a base optionally under inert atmosphere and reacting for at least 15 minutes at a suitable temperature, followed by washing with an ether to obtain the compound of formula I.

13. The process of claim 12 wherein the ether is TBME.

14. The process of any one of claims 12-13 wherein the suitable temperature is 15-25 °C.

15. The process of any one of claims 12-14 wherein the organic solvent is selected from an alcohol.

16. The process of any one of claims 12-15 wherein the base is selected from a base in a concentration sufficient to provide a pH of 12 or higher.

17. The process of any one of claims 12-16 wherein the base is sodium methoxide in methanol.

18. The process of any one of claims 12-17 wherein the reaction with a base is for at least 1 hour.

19. The process of any one of claims 1-18 wherein the molar ratio between the compound of formula IX and trimethyl((3,4,5-trifluorophenyl)ethynyl)silane is 5:4 to 1:3 and the organic solvent is in surplus.

20. The process of claim 19 wherein the molar ratio between the compound of formula IX and the catalyst is 20:1 to 2:1 and the organic solvent is in surplus.

21. The process of claim 19 or 20 wherein the molar ratio between the compound of formula IX and the base is 1:1 to 1:10 and the organic solvent is in surplus.

22. The process of any one of claims 1-21 comprising a step directly preceding step a)
(ia) reacting a compound of formula VIII 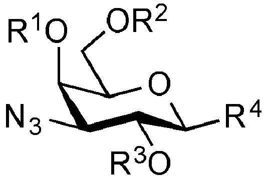 wherein R1, R2, and R3 are independently selected from protecting groups or hydrogen, provided that at least one of R1, R2 and R3 is a protecting group, and R4 is a halogen, with 5-bromopyridine-3-thiol and a base in a suitable organic solvent under suitable conditions, optionally under inert atmosphere, to obtain the compound of formula IX wherein R1, R2, and R3 are independently selected from protecting groups or hydrogen, provided that at least one of R1, R2, and R3 is a protecting group.

23. The process of any one of claims 21-22 the deprotonating agent is sodium bis(trimethylsilyl)amide.

24. The process of any one of claims 21-23 wherein R1, R2, R3 are all acetyl groups and R4 is as defined above.

25. The process of any one of claims 21-24 wherein R4 is chlorine.

26. The process of any one of claims 21-25 wherein the reaction takes place under inert atmosphere.

27. The process of any one of claims 21-26 wherein the organic solvent is selected from the group consisting of ethyl acetate, THF, toluene, DMF and acetonitrile, and mixtures thereof.

28. The process of any one of claims 21-27 wherein the suitable conditions in step (ia) are reacting a compound of formula VIII wherein R1, R2, R3 and R4, are as defined above, optionally under inert atmosphere and at a suitable temperature with 5-bromopyridine-3-thiol and the base in an organic solvent, and maintaining the reaction mixture at the suitable temperature, then continue the reaction for at least 15 minutes, and optionally isolating and purifying to obtain the compound of formula IX as a solid.

29. The process of claim 28 wherein the base is cooled to below room temperature before adding 5-bromopyridine-3-thiol over a suitable time and at a suitable temperature and followed by addition of the compound of formula VIII.

30. The process of claim 28 or 29 wherein the suitable temperature is below 25 °C.

31. The process of any one of claims 28-30 wherein the reaction is continued for at least ½ hours, at the suitable temperature.

32. The process of any one of claims 28-31 wherein the molar ratio between the compound of formula VIII and the base is 1:1 to 1:3.

33. The process of any one of claims 1-32 comprising a step directly preceding step ia)
(ib) reacting a compound of formula VII 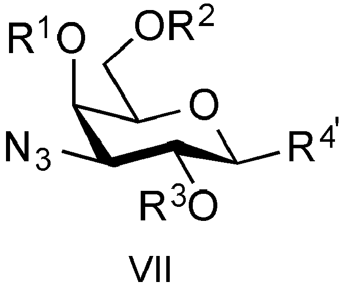 wherein R1, R2, and R3 are independently selected from protecting groups or hydrogen, provided that at least one of R1, R2 and R3 is a protecting group, and R4' is a SR5 or OR5 wherein R5 is selected from H, Z"-C₁₋₆ alkyl, Z"-C₁₋₆ alkenyl, Z"-C₃₋₆ branched alkyl, Z"-C₃₋₆ cyclo alkyl Z"-heteroaryl and Z"-aryl wherein Z" is SO, SO₂, C=O or C=S, with a reagent for activating the anomeric position for nucleophilic substitution, such as a halogenating agent or triflate, in a suitable organic solvent under suitable conditions to obtain the compound of formula VIII wherein R1, R2, and R3 are independently selected from protecting groups or hydrogen, provided that at least one of R1, R2, and R3 is a protecting group and R4 is a halogen.

34. A crystalline form of 5-Bromopyridin-3-yl 3-deoxy-3-[4-(3,4,5-trifluorophenyl)-1H-1,2,3-triazol-1-yl]-1-thio-α-D-galactopyranoside having formula (I).

35. The crystalline form of claim 34 which is a polymorphic form 1 as identified in the XRPD diffractogram in Figure 2.

36. The crystalline form of claim 34 which is a hydrochloride salt.
